Europäisches Patentamt

European Patent Office

Office européen des brevets

(11) Publication number: **0 289 667**
**A1**

## EUROPEAN PATENT APPLICATION

(21) Application number: 87302999.5

(22) Date of filing: 06.04.87

(51) Int. Cl.⁴: **C12N 9/02 , A61K 37/50**

(43) Date of publication of application:
09.11.88 Bulletin 88/45

(84) Designated Contracting States:
**DE FR GB**

(71) Applicant: **Nikken Foods Company Limited**
**723-1, Haruoka**
**Fukuroi Shizuoka(JP)**

(72) Inventor: **Ochi, Hirotomo**
**693-20, Haruoka Fukuroi**
**Shizuoka(JP)**
Inventor: **Katsukura, Yoshiteru**
**19-1, Asahigaoka 3-chome, Sendai**
**Miyagi(JP)**

(74) Representative: **Ablewhite, Alan James et al**
**MARKS & CLERK 57/60 Lincoln's Inn Fields**
**London WC2A 3LS(GB)**

(54) SOD active agent and method of manufacture thereof.

(57) An SOD (superoxide dismutase) agent comprises the cell preparation from strain of Streptococcus lactis potent in SOD and/or SOD-like activities, and a method of manufacturing the same consists of allowing such S. lactis to grow under the particular conditions on a particular culture medium. With said S. lactis cell preparation, the inhibitory effect is provided in senile changes such as fibrosis and calcification of cartilage matrix; aortic endothelial damage; hypertension; and atrophy of epidermis. A side-effect ameliorating agent that contains said S. lactis is provided for a superoxide producing chemotherapeutic drugs such as cis-platinum and adriamycin, and a radioprotective means is also provided by said S. lactis cell preparation.

EP 0 289 667 A1

## SOD ACTIVE AGENT AND METHOD OF MANUFACTURE THEREOF

### BACKGROUND OF THE INVENTION

#### Field of the Invention

The present invention relates generally to a medicament, and more specifically to the preparation of a useful medicine that takes particular effect as the senescence control agent, as an agent for ameliorating the side effect from the anticancer agent, and as means of protecting from exposure to any form of radiation.

#### Description of the Prior Art

The superoxide dismustase (which will be referred to hereinafter as "SOD") was discovered by McCord and Fridovich, both U. S. nationals, in 1969 as enzyme (EC:1. 15.1.1) that eliminates superoxide ($\cdot O_2^-$) by the dismutation. Since then, it has been found that $\cdot O_2^-$ and other active oxygen derived therefrom have some relationships to the biochemical reaction or biological changes that have been known so far. The 1965 Orgotein, which is obtained by extracting from bovine serum, is known to contain SOD as its active component and causes the anti-inflammatory effect (as disclosed in the British Patent No. 1,160,151 of Nov. 26, 1969 to W. Huber, Diagnostic Data Inc., and the U. S. Patent No. 3,637,640 of Jan. 25, 1972 to the same).

It is also known that aerobes such as E. coli and faculative anaerobes such as S. faecalis contain SOD, which can be induced in the presence of pressurized oxygen gas or superoxides (see "Medicine for Superoxides" by Ohyagi Yoshihiko, page 77, Kyouritu Publishing Co., 1981). Those are found in the intestine or stool for the warm-blooded animals. For E. coli, it may infect the urinary tract as an opportunistic infectious microbe. Some of its strains may produce toxin and cause haemolysis. For S. faecalis, it may also infect the urinary tract and may cause endocarditis. From the above fact, therefore, it is clear that they are not suitable sources for preparation as any form of oral administration agent that should desirably provide the SOD activity.

A number of studies have shown that the active oxygen such as superoxides contributes greatly to peroxidizing the fats for the living organism (such as the study by D. D. Tailor in the FEBS Letters 51 (1), 180-183, 1975). Another study by Huber et al. (W. Huber et al. in Proc. Int'l Meeting on Inflammation, Veorena, 24-27, Sept., 1979) has shown that the SOD agent, "Orgotein", which is derived from the bovine serum, can effectively control the peroxidation of the fats in the membraneous lipid by injecting it into the veins for the human or animal body, rather than by means of the oral administration.

So far, it has not been suggested that the preparation that may be obtained from the streptococcus lactis and which exhibits the SOD activity can effectively serve to ameliorate the side effects from the superoxide-producing chemotherapeutic agent. The next consideration is the sources of radiation, which may include ultraviolet rays that are contained in the solar radiation (particularly, the long-wave ultraviolet rays of wave lengths of 316 nm to 400 nm), the cosmic rays and the high energy X-rays used for the medical purposes, the $\gamma$-ray electromagnetic waves, and the high-velocity particle rays. There is quite a few possibilities that the biological organisms are exposed to those rays, which provide the ionizing action. As there are no conceivable threshold values above which the biological organisms might adversely be affected by those rays, their effect on the biological organisms should be tremendous. The accumulative effect of those radiations, even if it is the case with the ultraviolet ray, may affect the genes, possibly carcinogenic. The possible early effects of those accummulated radiations may include rash and pigmentation, and the possible later effects may include the wrinkled skins, accelerating senescene, and shorter life span. Although those effects are exerted on humans under normal conditions, no protective means is available at present. The exposure to the radiations even for the clinical purposes may adversely affect the fibrosis and hemogram, for which no adequate protection is provided.

It is thought that the effects of those radiations may be caused by their indirect action, that is, the action of free radicals that are generated when the radiations ionized water. Among others, the oxygen effect is the most important in that the cell membranes may be affected by the superoxide radicals generated by xanthine oxidase or the acetoaldehide oxidase, i.e., $\cdot O_2^-$, singlet oxygen $^1O_2$, etc. This means that it may cause any abnormal electrolyte equibirum outside and inside the cells, and inactivate the tissue. Particularly, the radiation anemia is caused by the haemolysis within erythrocyte.

The current inventors have discovered that S. lactis presents a high enzyme activity value of the superoxide dismutase (SOD), as compared with the other species that belong to the streptococcal group (such as S. faecalis, S. vobis, etc.), and has also made it clear that the S. lactis that does not inhabit in the intestine of mammals is easily digested through the gut and acts favourably on the free radicals within the living organisms, such as the action of reducing the serum lipoperoxides. The mechanism for generating those free radicals is known, wherein the aerobes absorb oxygen and through its metabolic process or exposed to any radiation, oxygen molecules are reduced by one electron into superoxide anions ($\cdot O_2^-$), or other active oxygens derived from the superoxide anions (such as singlet oxygen $^1O_2$, hydrogen peroxide, $H_2O_2$, and hydroxy radical, $\cdot OH$ oxidize the unsaturated fatty acids within the living organisms, thus the lipoperoxides result.

The superoxides play its role in the immunity action, in which they are emitted from the activated macrophage, favorably attacking the cancer cells. Similarly, there are some anticancer agents that effectively kill the cancer cells by producing the active oxygens.

Those anticancer agents include the Cis (III) platinum diammine dichloride which is usually known as Cis-platinum (which will be referred to as "Cis-DDP"), Streptonigrin, Breomycin Mitomycin-C, Adriamycin, Daunorubicin, and $\beta$-Lapachone. All of them are known as the superoxide producing chemotherapeutic agents (anticancer agents).

## OBJECTS OF THE INVENTION

One of the objects of the present invention is to offer an SOD-active agent that is remarkably effective for the following diseases.

(1) Liver cirrhosis, particularly similar lesions related to the aging, for which the said SOD agent has the inhibitory effect.

(2) Osteoarthritis that may occur frequently related to the aging, for which the said SOD agent provides the remarkable effectiveness.

(3) Atrophy of the skin primarily related to the aging, for which the said SOD agent has the preventive effect.

(4) Senile muscle degeneration related to the aging, for which the said SOD agent has the preventive effect.

In addition to the effects for the above diseases, the said SOD agent of the present invention provides the abilities to:

(5) enhance the various immune responses

(6) prevent aterioscrosis

(7) prevent hypertension

(8) modify biological response

Another object of the present invention is to provide an effective component that serves as the side-effect ameriolating substance for the superoxide-producing chemotherapeutic agent.

A further object of the present invention is to provide a radioprotective agent that contains the prepared S. lactis as its active component.

## SUMMARY OF THE INVENTION

According to the S. lactis of the present invention, any pathogenicity has not been recognized, and its toxicity test demonstrated safety. As its particular feature, the S. lactis as it is prepared as an oral administration purpose provides characteristic agreeable taste and flavor. Another feature is that it can easily be digested by lysozyme and therefore it will not proliferate (inhabit) within the intestine. This also provides the basis for justifying that the S. lactis is useful which cell wall is made of peptidoglycan. As it is clear from the foregoing description, the S. lactis may be used as it is, or may be sonically crashed, which may be used in large quantities through an oral administration. In either case, the S. lactis provides good taste and flavor and warrants a high safety. Thus, it has been made certain that it is the most suitable as the form of the SOD agent that has been described above.

It has also been demonstrated by using the S. lactis that it prepared to provide the SOD agent for the

0 289 667

aged rats and supplying it orally, that it can significantly inhibit the generation of lipoperoxide in the serum, liver, and brain for those rats, and is therefore useful as an anti-aging agent.

The inventor of the present application have discovered that the streptococcus lactis (which will be referred to as "S. lactis") usually contained in raw milk or dairy products, and vegetables such as corn, cabbage, lettuce and wheat, and has an SOD activity. It has also been verified that the product obtained by allowing it to grow on the culture medium can be used for the human being or animals without causing any safety or hygienic problems, and for the purpose of maintaining the good health, it may be used as an oral administration for a long time and can effectively inhibit the increase of the lipoperoxides within the living organisms. The present invention is based on the above discovery and verification.

The SOD active agent have the following characteristics:

(1) Nature/appearance/Property: The culture obtained from the S. lactis or its sonically broken product that has the freeze-dried powdery form offers its specific flavor, and has a good moisture absorption that helps it diffuse in water.

(2) General analysis:

    a) protein 57.31%

    b) fats 0.15%

    c) carbohydrate 15.77%

    d) ashes 22.52%

    e) water 6.60%

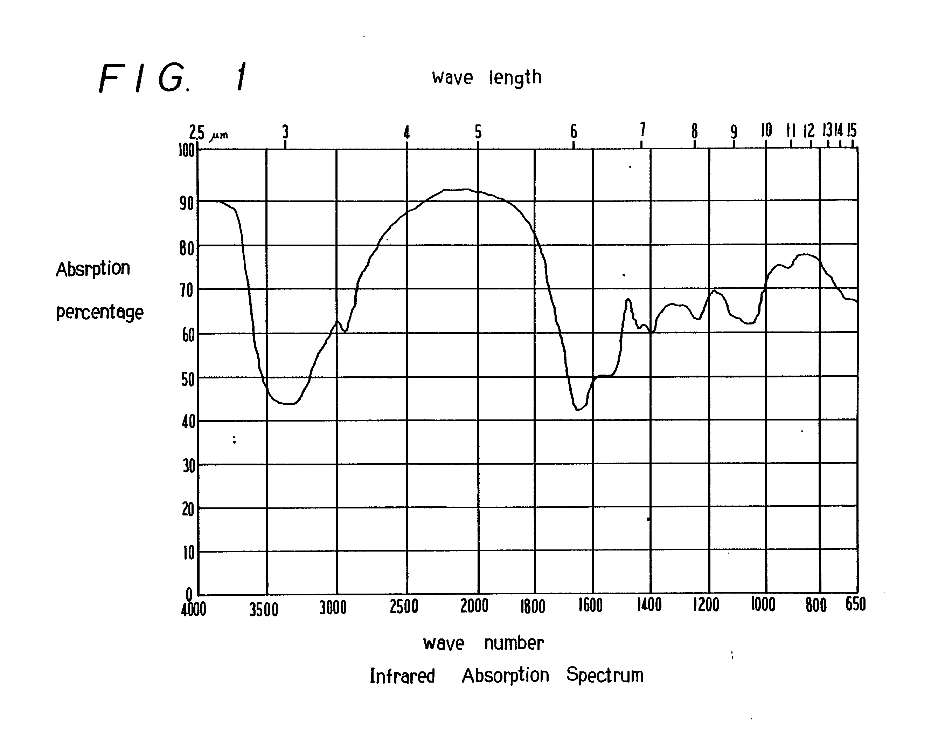

(3) The infrared absorption spectrum is shown in Fig. 1.

(4) Elementary analysis:

C 35.60%

H 6.44%

N 8.30%

S 0.27%

O 27.01%

Mn 28 mg/kg

Zn 160 mg/kg

Cu 6.6 mg/kg

Fe 260 mg/kg

(5) SOD activity: 4.5 units/g (S.lactis) based on the human SOD unit)

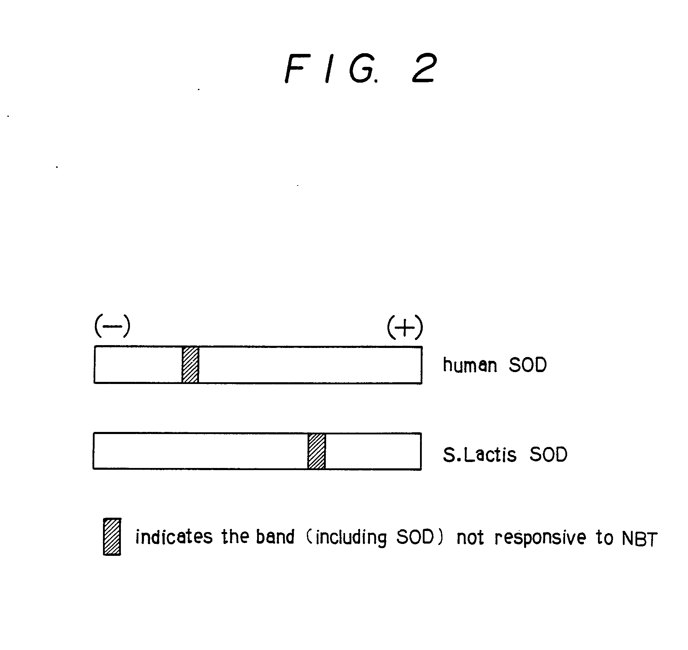

(6) Identification of SOD: It was isolated by using the polyacrylamide gel eletrophoresis, after which it was qualitatively determined by NBT (Nitroblue Tetrazolium) reaction (see Fig. 2).

(7) Identification of S. lactis: This was identified according to "Bergey's Manual of Determinative Bacteriology 8th ed., Williams and Wilkins, 1974 (see Table 1).

(8) Acute Toxicity: Single dose was given orally, and the observation was made to check for the acute toxicity for three weeks. Then, the autopsy indicated no such acute toxicity, LD50 of over 5 g/kg.

The bacteriological properties of the S. lactis used in the present invention are substantially the same as those described in the reference book mentioned in the above (7). That is, the S. lactis according to the present invention has the form of an oval diplococcus that exhibits no motility and no spore formation. Furthermore, it is Gram-positive, and has a final PH of 4.5 to 4.0 in acid production. It can coagulate litmus milk well. The optimum temperature is about 30 °C. It cannot grow at 45°C, but can grow at 10°C. It is also observed that it can grow on medium containing 4.0% salt, but it cannot grow on 6.5%. For the PH range in the alkaline side, it cannot grow at 9.6, but initiates at 9.2. It grows in 0.3% methylen blue milk.

The S. lactis of the present invention has the following physiological properties. That is, the ammonia production from arginine is positive, and neither hemolysin O nor S is produced. The fibrinolysin product is negative. Faculative anaerobe. It will not liquify gelatine. Decomposition of hippurate and esculin is positive, and the nisin production is negative or positive. The superoxide dimustase is positive, and when it is compared with the enzyme of human origin, it was 4.5 units per g of dried cells. The similar comparison was made between spieces of the streptococcus group and the SOD activity. The species that were used for the purpose of the comparison included streptococcus lactis (ATCC 19435), streptococcus faecalis (ATCC 19433), and sterptococcus bovis (Bergeys' Manual of Determinative Bacteriology, 8th ed., Williams and Wilkins, page 503, 1974). A 50 g of dried preparation from each species was provided. Then, each preparation was suspended in a physiological saline overnight. Then, it was sonicated at 20 KHz, 200 W for one hour, for example. The resulting filtrate was condensed to 10 times by a conventional method, and the condensed filtrate was dialyzed. The resulting dialysate was salted out by 60% saturated ammonium sulfate. The precipitate obtained by the salting-out was dialyzed by using a buffer solution of PBS to remove ammonium sulfate. Then, it was eluted by 0. 3 M sodium chloride through DEAE cellulose column,

4

the result of which was dialyzed by using the above buffer solution, and was freeze-dried. The final product was compared with a human superoxide dismutase sample (type III as supplied by the U. S. Sigma Company) with regard to the enzyme activity. The enzyme activity was 3,800 to 4,400 units/mg protein for S. lactis, 1,700 to 3,300 units/mg protein for S. faecalis, and 2,000 to 2,800 units/mg protein for S. bovis. The S. lactis exhibited the highest enzyme activity value. The in vitro test showed that the S. lactis was easily dissolved by lysozyme, and it was also digested in vivo in a very short time.

The study was made on the fermentation of sugars in terms of the acid production. The results was that it was positive for glucose, maltose and lactose, negative to positive for xylose, arabinose, sucrose, trehalose, manitol and salicin, and negative for raffinose, inulin, glycerol, sorbitol and starch.

It is clear from the above results that the S. lactis used as the SOD agent according to the present invention is substantially the same as those that have been described and known by this name, and that it makes potent SOD activity and can easily be dissolved by lysozyme and digested in vivo.

The cell preparation from the strain of S. lactis may be used as it is, or may be further processed by any breaking means (such as high-pressure, ultrasonic radiation, pulsating with beads), by autolysis or by enzymatic digestion. It is then freeze-dried. The resulting product remains stable for a long time at the room temperature and up to 37 °C with the SOD activity, and can be combined with auxiliary components such as vehicles, binders, diluents, $\beta$-carotene, and manitol. It may be administered per os in the forms of powders, particles, tablets, capsules, and syrup, depending upon the type of those auxiliary components. The amount of a single dose may vary appropriately, depending upon the age and weight of a patient. When it is administered per os, the daily preferred dose of the S. lactis preparation for an adult should usually be equal to 3 g to 60 g, which may be given one time or several times every day.

The conventional medicines as mentioned above may have their own favorable effect, but, on the other hand, may cause very serious side effect to the kidney, liver, heart, lungs, or bone marrow. It is now clear that those are caused by the superoxide. One typical example is the cis DDP that is known as the superoxide producing anti-cancer medicine. This medicine is known to be effective to testis cancer, bladder cancer, and ovarian cancer, but is also known to have a serious nephrotoxicity. As it is dose-dependent, its continued use is practically impossible. In order to avoid such nephrotoxicity, therefore, it is suggested that manitol, fulsemide, etc. be given with infusion. However, this suggested solution cannot prevent the nephrotoxicity (J. C. Gonzal et al., Cancer Treat Rep. 62, 693-698, 1978; B. R. Jones et al., Cli. pharmaco. Ther. 27, 557-562, 1980). The SOD was found to be useful for preventing the side effect by the cis-platinum and adriamysin (J. E. Mcginnes, Int. Symposium "Active oxygen and Medicine", Jan. 30-Feb. 1, 1979), but it still remains at the stage of the laboratory study. In this experiment, the route of administration is through the i.v. injection, making it difficult to manufacture an SOD agent economically and technically.

As the background of the present invention has been described, the demands are increasing for an economical SOD agent that provides an preventive means for patients who take the superoxide producing therapeutic agents and is commercially available as an oral administration to those people.

The present invention is based on the fact that the cell preparation from the strain of streptococcus lactis potent in the SOD activity can effectively inhibit any possible nephrotoxicity when it is given as an oral administration to a mouse which took the cis-platinum.

For the conventional lactic culture, the bacteria is supposed to be resident in the human intestinal tract, therefore orally administered, it is allowed to grow in the intestine. The expected effectiveness of it is thus resulted.

As contrasted with those conventional preparations, the S. lactis preparation according to the present invention uses the type of lactic bacteria that is non-resident in the intestine but is only used for dairy products such as cheese, yogurt, etc. Whether the preparation involves the breaking process or not, it can easily be digested in the animal intestine, and may not be expected to remain, grow and increase within the intestine but can effectively ameliorate the side effect of the aforementioned chemotherapeutic agent. Thus, the chemotherapeutic agents that are expected to appear in a varieties can be used effectively and smoothly. In addition, it may be possible that the S. lactis will be included in the regimen for one of the most incurable diseases, or cancer. By doing so, it may be expected that this will contribute greatly to the increased cure percentage or therapeutic ratio for the cancer.

S. lactis preparation:

S. lactis is allowed to grow, for 24 hours at 37 °C on a static aqueous culture medium that contains 1% glucose, 0.8% pepton, 0.4% yeast extract, 0.3% sodium citrate, and 0.25% sodium bicarbonate. Then, the culture is subjected to the centrifugal separation at 10,000 rpm for ten minutes. It is washed in the physiological saline three times, and then is sonically broken. The broken product is then freeze-dried, and is used for the experimental purpose. The polyacrylamide gel electrophoresis technique as offered by Fridovich et al. (Beauchamp, C. and Fridovich, I (1971) Anal. Biochem. 44. 276-287) is used to check to see that the product contains the SOD, and the human SOD sample (type III as supplied by the U. S. Sigma Co.) is used to determine the SOD activity by means of the NBT method. It has been found that the SOD activity is 4.5 U/g dried S. lactis preparation.

Side-effect ameliorating agent for the chemotherapeutic agents:

The side-effect ameliorating agent contains the S. lactis preparation potent in the SOD activity as its effective component, and may contain auxiliary components as required for the preparation, such as vehicles, binders, diluents, and others (such as $\beta$-carotene, manitol). It may have any form of powders, granules, tablets, capsules, or syrup, depending upon the type of the auxiliary component used. It may be provided as an oral administration agent. The amount of a single dose may vary depending on the age and weight, and 3 g to 60 g of S. lactis preparations per day is preferred for an adult.

One preferred form of the present invention is provided as a single unit of dose that may be used as divided one or several doses per day.

About the problem of ameliorating the side effect for the chemotherapeutic agents

It has been mentioned that the inventor of the present application discovered that the cell preparation from the strain of the streptococcus lactis (S. lactis) potent in the SOD activity can significantly reduce the value of the lipoperoxide in the serum, liver and brain for a rat which suffers senile changes as it grows older. The inventor also considered that it would provide an effective preventive means to biological changes associated with the exposure to radiations that turn out to cause the rapid senile changes. The inventor studied this problem, and found that it was also effective in this respect. The present invention is also based on this discovery.

Brief Description of the Drawings

Other objects, features and advantages of the present invention may readily be understood from the detailed description of several specific examples that follows hereinafter with reference to the accompanying drawings, in which:

Fig. 1 is a diagram that represents the Infrared absorption spectrum for the S. lactis preparation;

Fig. 2 is a diagram that represents the polyacrylamide gel electrophoresis pattern for the S. lactis SOD;

Fig. 3 is a microscopic photograph showing the forms of living cells, in which "a" represents untreated S. lactis, and "b" represents lisozyme-treated S. lactis;

Fig. 4 presents the comparison of the lipoperoxide in the serum, liver and brain for a rat which has aged twenty-seven months;

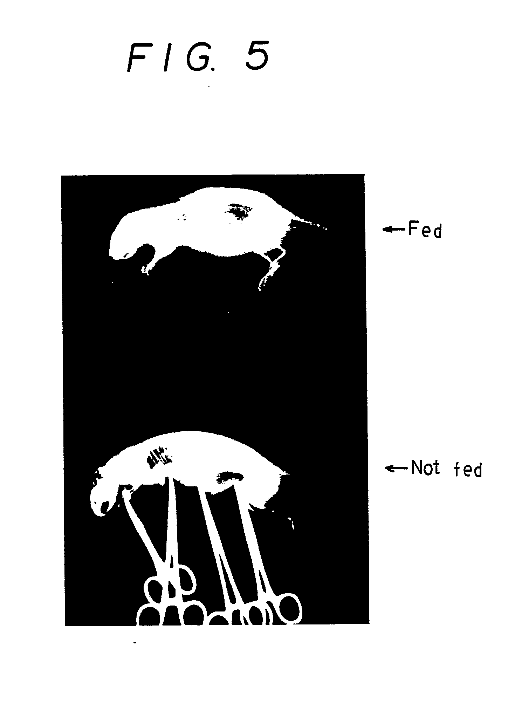

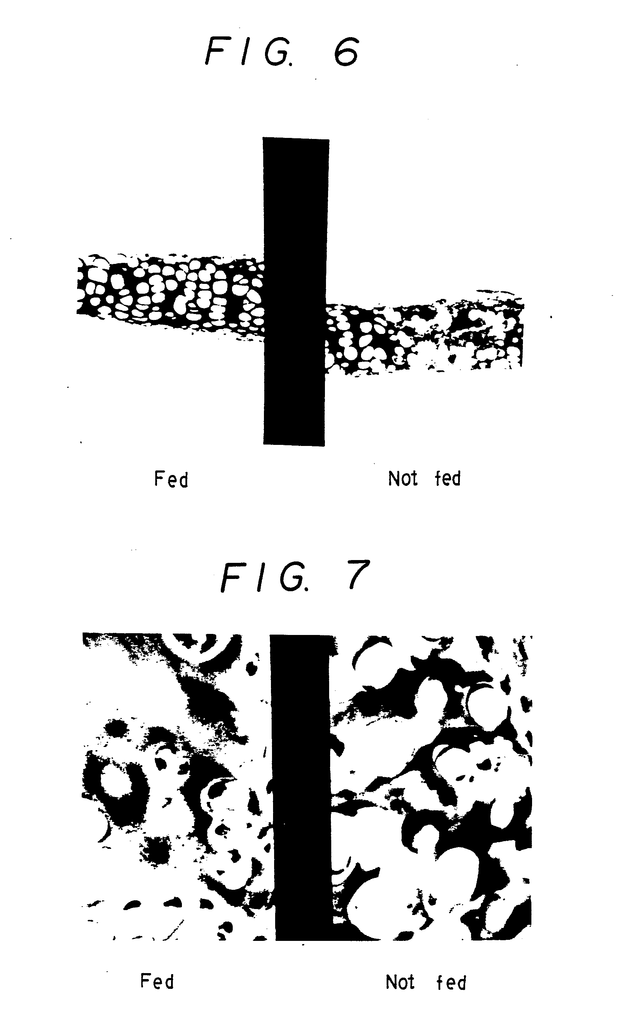

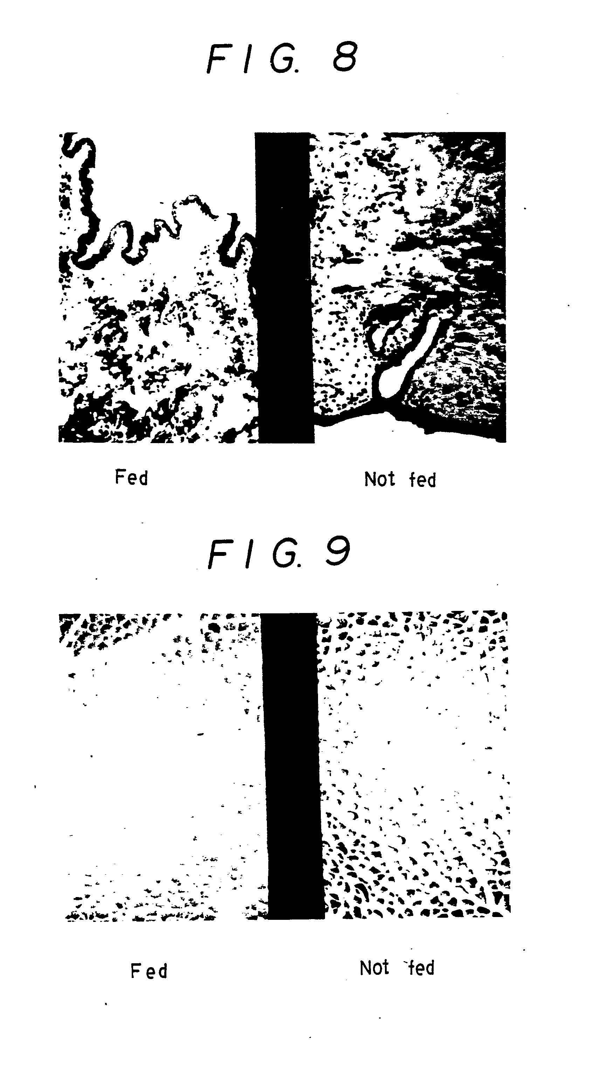

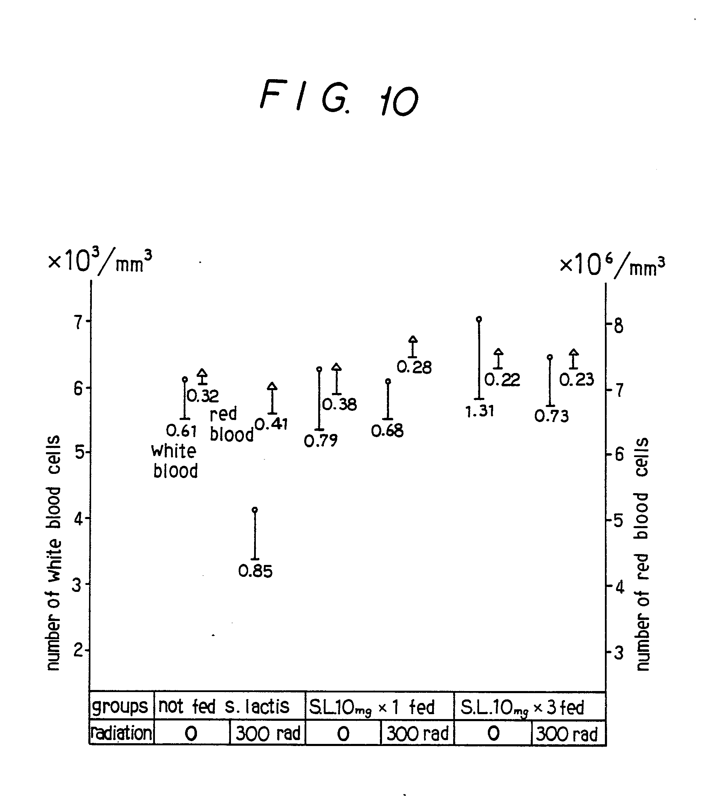

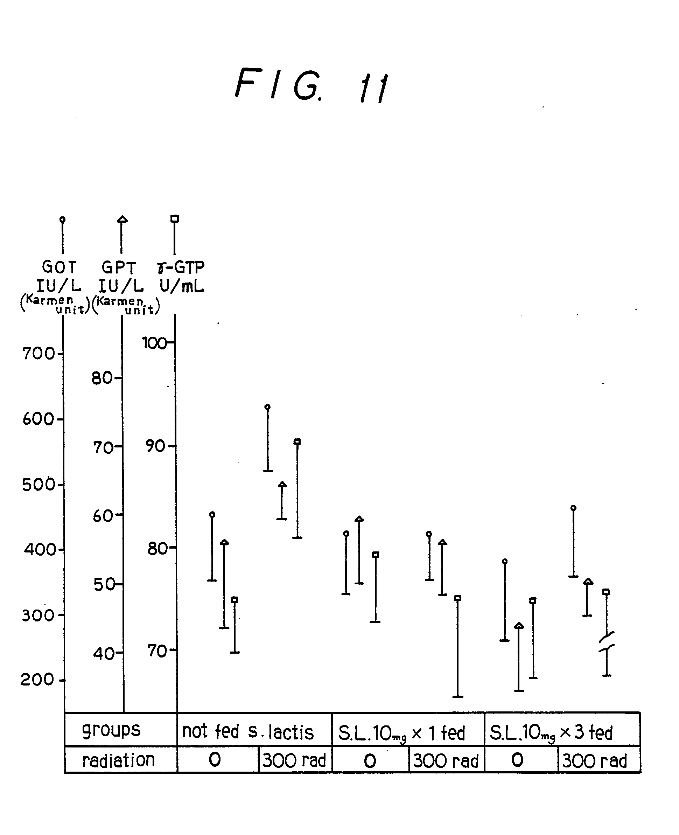

Figs. 5 through 9 are photo pictures showing the results obtained by comparing the two different rats, wherein Fig. 5 is an X-ray picture showing the differences in the bone between one rat that was given S. lactis and the other which was not, Fig. 6 shows the differences in the xiphoid cartilage between the one and other rats, Fig. 7 shows the different dynamic behavior of the interstitial calcium of cartilage between the two different rats, Fig. 8, shows the differences in the epidermis between the rats which have aged 27 months, of which one was given S. lactis and the other was not given, Fig. 9 similarly shows the difference in the greater psoas muscle, Fig. 10 is a diagram which shows the radioprotective effect provided by the S. lactis preparation (that is, the effect on changes in leucocytes and erythrocytes), and Fig. 11 is a diagram which shows the radioprotective effect provided by the S. lactis (that is, the effect on changes in serums GOT, GPT, and $\gamma$-GTP).

Details of the Preferred Embodiments

Several preferred embodiments of the present invention will be described in further detail, followed by the description of the corresponding experimental cases.

(Example 1)

A culture medium that is chosen to allow the S. lactis to grow consists of 1% glucose, 0.8% pepton, 0.4% yeast extract, 0.3% sodium citrate, and 0.25% sodium bicarbonate. The culture medium is sterlized and cooled, on which 3 to 10% of S. lactis is implanted as a starter. This starter is allowed to grow statically at 37°C for 24 to 30 hours. The resulting culture is then subjected to the centrifugal separation that occurs at 10,000 rpm for 10 to 30 minutes. The cultured S. lactis is obtained. This S. lactis is then washed with a physiological saline three to five times, after which it is exposed to radiation from the continuous ultrasonic radiation source. It is thereby broken. Immediately following this, the broken S. lactis is freeze-dried by the appropriate freeze-drier. The product contains 0.375 to 0.5 kg of dried S. lactis preparation per 100 g of the culture medium.

Part of the S. lactis obtained above by the supersonic processing is further subjected to the centrifugal separation that occurs at 15,000 rpm for 60 minutes. The supernatant portion produced by the centrifugal separation process is condensed to ten times by the conventional method. The result is placed in a cool environment for 24 hours, and is dialyzed by using a physiological saline buffer solution (PBS). The resulting dialysate is freeze-dried, the result of which is used as a sample of an enzyme preparation.

As an alternative method, after the S. lactis was removed from the culture medium and washed, part of the S. lactis that remains intact is immediately freeze-dried, and the freeze-dried S. lactis is impregnated in an ether so that the plasmolysis can occur. The result obtained by the plasmolysis is allowed to autolyze in a 5 to 50 °C and PH 5 to 9.2 water. The product thus obtained is then subjected to the centrifugal separation that occurs at 15,000 x g for 60 minutes, and the resulting supernatant portion is removed, which is used as a enzyme preparation sample.

(Example 2)

An aqueous culture medium that is prepared in the usual manner consists of 1% glucose, 0.8% pepton, 0.4% yeast extract. 0.3% sodium citrate, and 0.25% sodium bicarbonate. The S. lactis according to the present invention is allowed to grow without agitation on the culture medium at 37°C for 24 hours. The resulting culture is then subjected to the centrifugal separation that occurs at 10,000 rpm for ten minutes. The product obtained during the centrifugal separation process is washed with a physiological saline three times, and is subject to the freeze-drying process. A sample that is taken from the product contains an SOD activity of 4.5 U/g S. lactis preparation.

The radioprotective agent obtained by the above steps contains (as its effective component) an S. lactis preparation potent in the SOD activity, and may optionally contain any auxiliary components that are required for the preparation, such as vehicles, binders, diluents and the like. This agent may be provided in the form of powders, granules, tablets, or injection, depending upon the type of the auxiliary components used. Furthermore, it may be used as an oral administration or otherwise. The amount of a dose may vary depending upon the age and weight, and its preferred amount per day for an adult may be in the range of 3 g and 60 g, when it is used per os.

In the preferred embodiment of the present invention, the amount of a dose that is given every day may have the form of a unitary administration that may be given over one or more times per day.

The following description provides several experimental cases that have been made to check the validity of the corresponding samples obtained according to the above described embodiments.

(Case 1)

In this case, the experiment has made to check for the presence of the S. lactis SOD by using the electrophoresis techinique and to determine the SOD activity.

(1) The polyacrylamide gel electrophoresis method, which was reported by Fridovich et al., is used to check the presence of the enzyme preparation sample obtained in the preceding examples. The gel obtained after the electrophoresis process is then immersed in 2.45 mM NBT (Nitroblue Tetrazolium) solution for 20 minutes, and is further immersed in a PH7.8 mixture that consists of 28 mM tetramethylethylenediamine, 0.028 mM riboflavin, and 36 mM potash phosphate for 15 minutes. The gel that has been processed above is then moved into a test tube in which it is exposed to the radiation with a 15 watt-fluorescent light for 30 minutes. Thus, the superoxide anion is produced by reducing the riboflavin by light, and reduces the NBT. Areas other than those where the SOD activity exists are colored blue-purple, from which it has been determined that the SOD is present (Fig. 2).

(2) The NBT method was used to determine the SOD activity:

The standard curve was obtained by diluting the human SOD (Type III, U. S. Sigma Co.) 3,000 u/mg protein with a phosphate buffer solution, and then preparing the diluents of x 10, x 100 and x 1000 (Imanari, Toshio et al., "Igaku no Ayumi" 101 496-497, 1977). A reaction mixture is composed of 2.4 ml of 0.05 M $Na_2CO_3$, 0.1 ml of 3 mM xanthine, 0.1 ml of 3 mM bovine serum albumin, 0.1 ml of 0.75 mM NBT, and 0.1 ml of SOD sample solution. In ten minutes, 0.1 ml of xanthine oxidase solution is added, the mixture is allowed to stay at the room temperature for 20 minutes. Then, 0.1 ml of calcium chloride is added for stopping the reaction. At this time, the reading of O. D. at 560 nm is made, and the SOD unit is calculated from the standard curve. From this calculation, the SOD activity (specific activity) per g of dried S. lactis is determined.

(Case 2)

The following describes the results of the testing that has been made to check the safety of the S. lactis according to the present invention.

The cell preparation of S. lactis obtained by allowing it to grow on the culture medium and whose SOD activity was determined in terms of its qualitative and quantitative properties, according to the steps described in the preceding embodiments, was used for Wistar rats (which included male and female rats, each aged 15 weeks and weighing about 250 g). The testing for those rats has occurred in different routes such as oral administration, i.v. injection, and intra-peritoneal injection, to check for acute toxicity (three weeks), sub-acute toxicity (three months), and chronic toxicity (six months and 24 months). The results of the toxicity testing for each case are shown below.

(1) Acute toxicity

(a) Oral administration: F2 solid feeds (supplied by Funabashi Farm) was used as basal feed. The S. lactis preparation was put in 2 ml of sterlized physiological saline and provided through stomach tubing with 1, 10, 100, 1,000 and 5,000 mg for each group of rats. Then, each of the S. lactis suspensions was given to the corresponding group of rats by the single oral administration per day, and only the physiological saline was given to the control group of rats;

Each group of rats included five rats. For each group, the observation continued for three weeks since the oral administration was made. The autopsy that occurred following that observation showed no abonormal changes in the weights of those rats. No death case was noted. The macroscopic examination of the hemogram and organs indicated there were no changes as compared with those for the cases in which only the physiological saline was given. It may be estimated from the above data that LD50 is above 5g/kg.

(b) i.v. injection: As for the oral adiminration, male and female rats were used, and the S. lactis preparation was put in 2 ml of physiological saline and provided with 1, 2, 10, 100, 500 and 800mg for each group of rats. Each of the S. lactis distributions was given to the corresponding group of rats by means of the tail i.v. injection. Since then, the observations continued for three weeks. The weight changes for those rats which received the i.v. injection occurred in the same manner as for the reference rats chosen for the comparative purpose. The weight increased to between 10 and 15 g per week. No death case was found, but the rats which received 500 mg and 800 mg of S. lactis, respectively, suffered shocks immediately after the injection. Those shocks lasted less than one hour, and thereafter those groups showed no difference from control.

(c) Intra-peritoneal injection: The S. lactis preparation was put in 2 ml of physiological saline, and i.p. injected with 1, 10, 100, 500, and 1,000 mg for each group of rats. The observation continued as for the preceding cases. The results were that three rats out of five in the 500 mg group and all of the five rats in the 1,000 mg group died in 24 hours. The autopsy showed that they suffered from fatal splenoma. It may be estimated from the above data that the LD50 for the intra-peritoneal injection is below 100mg per rat.

(2) Sub-acute toxicity

(a) Oral administration: Each group of rats included five male and female Wistar rats of 10 weeks of age. Basal powdered feeds F2 (supplied by Funabashi Farm) were used, to which the S. lactis preparation was added so that 200, 1,000 and 15,000 ppm of the S. lactis could be contained and then pelletized. For the control group, the above basal feeds were supplied in their pelletized forms. Each group was fed ad libitum the respective solid feed and water, which continued for three months. During those months, each rat took the average 15 to 20g each day. The daily observation as well as the autopsy that occurred after the three months showed no changes in histochemical examination.

(3) Chronic toxicity

This testing occurred in the same manner as for the sub-acute toxicity testing, except that each group of rats included five female Wistar rats of 5 weeks of age, and two feeding periods, 6-month and 24-month, were set up, and after each of the periods, the autopsy followed by the histochemical examination. Each of the examinations indicated no abnormalites that may have been caused by the S. lactis preparation.

It may be concluded from the results of the above observations that occurred to check the various levels of toxicities that the S. lactis preparation may be fed without causing any safety problems.

(Case 3)

The following describes how the digestion occurred for the S. lactis according to the present invention, in order to demonstrate that it can be easily digested.

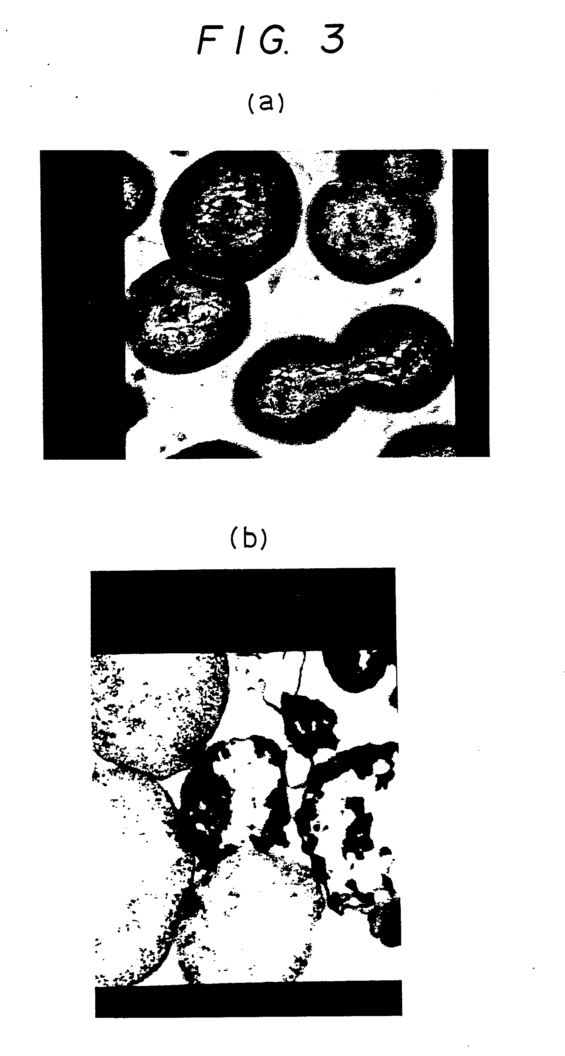

The digestion testing was made to check to see how the S. lactis preparation which had entered the digestive system would go through the digestion. Since it is known that the membrane of the S. lactis is composed principally of peptidoglycan, the in-vitro examination in which lisozyme was used. During this digestion testing, it was observed that the cell walls disappeared completely in two hours (see Fig. 3), and the plasmolysis occurred, after which the cell component leaked out of the membrane soon. Then, the in-vivo digestion testing occurred for three couples of male and female Wistar rats (of 5 months of age) which were fed sterlized feeds. A preliminary step was taken before the S. lactis preparation was given to those rats, during which they were allowed to live on sterlized water alone for ten hours. The S. lactis preparation was placed in 5 ml of sterlized physiological saline, and the resulting suspension that was equal to lg (by dry weight) was then given to the rats by the forced oral administration. After two hours, four hours, and ten hours, under the Nenbutal anesthesia (Nenbutal is the registered trademark) each pair of rats were sacrificed. Then, jejunum and ileum were completely removed from each of the rats, and the sampling occurred in the usual manner where 5 mm-long samples were obtained for each 2 cm-length of the removed jejunum and ileum. Those samples served as the opto-microspcopic and electronmicroscopic specimens. Two hours after the oral administration, it was observed that the S. lactis that entered the intestinal tract remained almost intact. After four hours, it was observed that substantially one-fourth of the S. lactis had partially been digested. After ten hours, it was observed that substantially all of the S. lactis had gone through the phases of the digestion process, presenting some or other changes. It was also observed in parallel with the above observations that the digested portion of the S. lactis had been taken in by phagocytosis of the Panet's cells within the intestinal crypta. More specifically, the observation indicated that vacuoles were formed in the Panet's cells and lisozyme connected with the vacuoles produced various enzymes, and that those enzymes, particularly the lisozyme, helped the S. lactis digested and dissolved in the vacuoles. As the S. lactis was thus digested and dissolved, it was observed that it was being stored into the Panet's cell or being emitted therefrom.

As described above, it was made clear that when the S. lactis was given per os, it could be digested very efficiently, some of which could be absorbed through the Panet's cells within the intestinal tract.

(Case 4)

In this case, the S. lactis was allowed to grow on 50 kg of culture medium, which contained 1% glucose. 0.8% pepton, 0.4% yeast extract, 0.3% sodium citrate, and 0.25% sodium bicarbonate. For the purpose of the culture, the culture medium was sterilized and 10% of starter was added. The medium was kept warm at 37° C for 24 hours, and then was subjected to the centrifugal separation for 15 minutes at 10,000 rpm. To the S. lactis that was concentrated during the centrifugal separation process was added a sterlized physiological saline whose amount was equal to substantially ten times that of the concentrated S. lactis. Again, the centrifugal separation occurred for the result in the same manner as the first one, and was repeated for another. At the finish of the centrifugal separation process, RIBI Cellfractionator (RF-1 Sorval) was used to break the culture under the applied pressure of 9 kg/cm2, which was repeated several times. Immediately after this, those were freeze-dried, from which 5.1 unit/g of SOD activity was obtained.

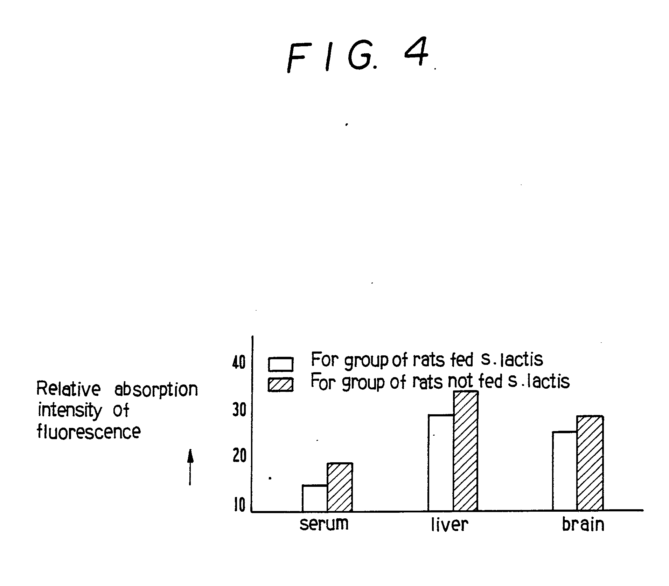

Two different groups of rats were used for the purpose of testing the above-obtained S. lactis preparation, each of which included ten female Wistar rats which were initially aged 10 weeks and were kept fed with the particular feeds as specified below until they were aged 27 months. Under the above conditions, the rats in the one group were only fed the reference compounded solid feeds F2 (as supplied by Funabashi Farm), and for the other group rats, a mixture that was composed of the above compounded solid feeds and the S. lactis preparation which were mixed to a concentration of 10,000 ppm was reached was provided and given in the form of pellets. Each group of rats had been fed in that manner until they grew to be 27 months old, when they were killed. The comparison was then made between the two different groups by using thiobarbituric acid fluorescence absorption spectroscopy in terms of the amount of lipoperoxide (relative absorption intensity of fluorescence spectroscopy) in the serum, liver and brain (see Fig. 4). For the control group of rats, the serum lipoperoxide was nearly two times as high as that for the study group of rats, and the lipoperoxides in the liver and brain were also significantly high. Other examinations that were made from the pathological and histological aspects showed that, for the control group, there were noticeable symptoms associated with the aging, such as those which appear as the superoxide increase, including the decrease in the cyclic GMP in the serum. The alopecia and kyphosis, which can readily be identified in appearance, appeared for all the rats in the control group, whereas for study group, no such case occurred for any rat.

(Case 5)

The following description is provided to present the results of the experiment that occurred to check for the preventive effect of the present invention against the liver cirrhosis.

The possible causes for the liver cirrhosis may be classed into those which are associated with virus, alcohol, and metabolism, according to the types thereof. It may also be caused by the aging.

As the pathological examination shows, the liver cirrhosis appears as the damaged liver cells (necrosis and abnormal nucleus), the fibrosis of the hepatocyte, and the generalized occurrence of the lipid droplets, all of which are common to all the types of the causes. During the animal experiment that took place for the Wistar rats that were fed for a long time, it was also observed that there were the damaged liver cells, the cell infiltration associated with the fibrosis of the hepatocyte, and the occurrence of the lipid droplets. There is no effective therapeutic means that could remedy or prevent such liver cirrhosis, particularly that which may be caused by the aging.

Then, the following description illustrates how the S. lactis strain prepared by the present invention can effectively prevent the occurrence of the liver cirrhosis due to the biological changes associated with the aging in particular.

Each group of 27 female Wistar rats aged 10 weeks was employed as one group containing the rats which were to be fed the basal feeds F2 to which 10,000 ppm of the S. lactis preparation was added and the other group containing the rats which were fed the basal feed F2 alone. At the first week, at 6 months of age, and at 27 months of age, the rats in each of the groups were killed, for which the autopsy took place. Those rats were examined pathologically and histologically in order to check for the damaged liver cells, abnormal nucleus, lipid droplets, and the cell infiltration associated with the fibrosis of the hepatocytes, and the measurement was made of the serum collagen (as well as the hydroxyproline that has the parallel relationship with the serum collagen).

The results are given in Tables 1 and 2 below. The opto-microscopic observation, which took place for the group of rats which had been fed the basal feeds alone and aged 27 months, shows that in all cases there were medium fat deposition, periportal fibrosis, inflammatory cell infiltration into the hepatic lobules, liver cell chord disorder, and increase in the contracted liver cells. For the other group of rats aged 27 months, no organic changes were observed as for the same rats aged six months. The electron-microscopic comparison that was made between the two groups shows that for the control group, the mitochondria were usually swollen due to the edematous change, and there were many high electron density metallic salts at the cristae and matrix. Many vacuoles were found in the intima (including visicle) and the growth of collagen could readily be found in the space of Disse. From the biochemical aspect, the collagen in the vessel has the parallel relationship to the hydroxyproline, and presents a high significant value for the study group. From the above results, it has been determined that the S. lactis preparation according to the present invention contains a useful compnent that can effectively prevent the occurrence of the liver cirrhosis.

Table 1.  Comparison in the liver changes for the two different groups:

| Group | Check Items | 1st. Week | 6 months | 27 months |
|-------|-------------|-----------|----------|-----------|
| Control | N | − | + | 4+ |
|         | F | − | 2+ | 3+ |
|         | C | − | + | + |
| Study | N | − | + | + |
|       | F | − | + | + |
|       | C | − | + | + |

Notes:

N:  necrosis and abnormal nucleus for liver cells

F:  lipid droplets

C:  cell infiltration into the fibrosis

−:  no changes observed

+:  slight

2+:  moderate

3+, 4+:  five different samples were used for each of the five stages from − to 4+

Table 2.  The content of hydroxyproline ( $\mu$g/DNA $\mu$g) in the aorta and mesenteric aorta:

| Group | 80 days of age | 6 months of age | 27 months of age |
|-------|----------------|-----------------|------------------|
| Control | 1.08 ± 0.06 | 1.65 ± 0.08 | 5.32 ± 0.64 |
| Study | 1.80 ± 0.08 | 1.75 ± 0.12 | 3.93 ± 0.03 |

Note:  Each value that appears in the table represents the average value with the standard erros (S.E.) ($P < 0.01$).

(Case 6)

In this case, the experiment was made to check how effective the S. lactis according to the invention would be for the spondylosis deformans. The spondylosis deformans may basically be characterized by the deformations in the disk, which include the projection of the disk and the osteophyte formation in the pyramide border. The typical symptoms for the spondylosis deformans include pains, root signs, and spinal signs. In general, this occurs more frequently for adults aged over 40 years, and particularly, the spinal signs occur with the highest incidence. It is also noted that the lumbago that appears as the principal sign of the spondylosis deformans manifests itself as a result of the deformed disk, cracked fibre-rings, osteophyte formation, and expanded bands and other organisms. It takes place during relatively early years for heavy-labor workers or farmers. In general, however, it is associated with the aging, in which case deformation is caused in the physiological spinal curvature. The deformation occurs such that the kyphotic thoracic vertebrae is developed into the upper and lower parts and is increased, while the lumber flexure is decreased. As a result, the cervical vertebrae in turn is increased, and the slant of the basin is decreased. This takes place as the rounded back, which is particular to the aged people.

As precautions to be taken against this rounded back, it is suggested that an excessive load on the backbone should be avoided, or efforts should be made to prevent the shrinkage in the bones or reduction in the muscles by taking the proper posture or taking any adequate physical excercise or working moderately. There is no positive means, however, which could effectively prevent this.

As for the preceding testing that occurred to the check the effectiveness of the S. lactis for liver cirrhosis, the Wistar rats were used for the purpose of checking its effectiveness for the spondylosis deformans. Those rats are classed into two groups, one group consisting of rats which are to be fed with S. lactis preparation (study group) and the other group consisting of rats which are not to be fed with the S. lactis preparation (control group). At the age of 27 months, the two groups were compared. The results were that for the control group, the alopecia and kyphosis were observed in all cases. For the study group, on the contrary, they were not found in any of the rats. When all of the rats in both groups were examined by obtaining the X-ray image, it was found that the rats in the study group were all normal, whereas there were abnormal bones for the rats in the control group, which indicates the kyphotic thoracic vertebrae (see Fig. 5).

The next step that was taken was to check for any senile changes in the cartilage texture. For this purpose, the chelate reagent by GHA (Kashima, H. K. Stain Technol. 41, 49-55, 1966) was instrumental to look into the dynamic behaviour of the calcium within the living organisms. Then, the distribution dynamics of acidic glycoprotein was checked by using the Diamin-Alcian-Brue method and by using the dye-dissociation method that relies upon the ion intensity by the magnesium chloride (Spicer, S. S., J. Histochem. cytochem., 13, 211-234, 1965). Then, the study of any changes in the xyphoid cartilage was made by using the ordinary staining method (H. E., PAS-AB, Van Gieson). The results of the observation for the cartilage are that for the non-S. lactis fed group (control group), there was fibrosis in the stroma and the calcium salt was deposited like sands in all cases (Figs. 6 and 7). It was also found that chondrosulphate in the cartilage matrix generally changed to B-type, kerato-type, whereas for the S. lactis fed group (study group), although some fibrosis and limes were found, the chondrosulphate was mostly C-type, which occupied the entire area of the matrix (Figs. 4 and 5). The distribution of the chondrosulphate that was obtained when the rats were aged 27 months is presented in Table 3, which clearly shows the difference in the cartilage hardening between the two control-study groups.

From the above results, it may be said that the S. lactis preparation of the invention provides a useful, effective means for preventing the occurrence of the spondylosis defomans associated with the aging.

Table 3.   The types of chondrosulphate that were found in the cartilage for the control-study groups at the age of 27 months (as compared by the Alcian-Blue mehtod at PH of 1.0 after the enzyme dissolving):

| Enzyme hydrolysis | chondro-itinaseABC | | chondro-itinaseAC | | no treatment | | matrix chondrosulphate distribution |
|---|---|---|---|---|---|---|---|
| Group | C | M | C | M | C | M | |
| Control (Ce) | ±～ l | ～ | l ～+ | −～+ | ++ | ++～+++ | generally B type, or kerato type |
| Study | + | + | + | + | ++ | ++ | most C type |

Note:  C:      cartilage cell surface,    M:  matrix,

±～ l:  weak reaction,          +:  reacton positive,

−:      reaction negative,

++～+++:  strong reaction (as observed for ten rats in each group)

(Case 7)

In this case, the experiment was made to check to see how effective the S. lactis preparation of the invention would be in preventing the occurrence of the atrophia of skin, the results of which are described below.

The symptoms for the atrophia of skin may include the thinner and softer skins, the minute wrinkles, the brighter skins, the removed hairs, and the degradation in the functions of the sebaceous glands and sweat glands. The causes and etyology for those symptoms are not clarified, but the atrophia senilis that may appear as a sign of senescence associated with age is the physiological atrophia that occurs in the generalized manner. When this occurs, the epidermis becomes a single layer, and the collagen beneath the epidermis will be swollen and broken, with the accessory organs (sebaceous glands and sweat glands) disappearing.

For the comparative purpose, the experiment took place by feeding the two groups of rats for 27 months after weaning in the same procedures as for the preceding cases.

For the epidermis (dorsal skin), the results show that for the nonfed group (control group), the dermic collagen fibre was cut into short pieces, and was swollen. It was also found that coupled with the blood plasma infiltration that occurred, there was the so-called fibrinoid degeneration. Furthermore, the accessory organs such as sebaceous glands, etc. disappeared, which could readily be identified (fig. 8). The epidermis was found to have become a single, flattend skin layer, presenting alopecia. For the group of rats which had been fed the S. lactis, on the contrary, it was found that the reticulin had been developed into the epidermis, and the dermic collagen fibre was not broken, as was the case with the 6-month aged rat group.

From the above results, it may be concluded that the S. lactis preparation of the invention also contains a useful component that can effectively prevent the occurrence of the atrophia of skin associated with the aging.

(Case 8)

The senile-amyotrophy-preventing effect of this agent will be described on the basis of the following experiment.

The incidence of senile amyotrophy, muscular atrophic changes resulting from aging, which is accompanied by abnormal changes is significantly high for persons aged over sixty. This disease is characterized by a fibrous ring which consists of other denatured fibers surrounding muscule fibers. At present, the cause of this muscular atrophy resulting from aging is unknown and there is no preventive measures for this disease.

In the same way as in the aforementioned extended period administration test on Wistar rats (until they become 27-month old), we compared a group administered with this agent (study group) with a nontreated group (control group).

In the control, atrophy of skeletal muscle fibers (Fig. 9) and infiltration of chondroitin sulfate of the B-type among skeletal muscle fibers were observed. It was found that the substances contributing to these changes had easily chelated with a calcium salt exhibiting tissue sclerosis. Similar changes were only rarely observed in any sample collected from the subjects of the group administered with this agent.

The above results indicate that this agent is effective and useful for preventing senile amyotrophy.

(Case 9)

The immunological-competence-enhancing effect of this agent will be described on the basis of the results of the following experiment.

We determined the immunological-competence-enhancing effect of the microbial cell preparation of this invention by the number of splenic cells forming hemolytic plaques.

Five to six week old female mice of the C3H/HeSLC species (SPF) were used as test animals. Each of nine animals was once administered with 0.5 ml of a suspension of the S. lactis cell preparation in isotonic sodium chloride solution (10 mg of the microbial cell preparation /mouse) by using a stomach tube (study group). We compared this group with a nontreated group consisting of the same number of mice (control group) by the following method. Sheep red blood cells (SRBL) stored in an Alserver liquid was washed with phosphate buffer three times immediately prior to use. After 0.2 ml of a 25% SRBC suspension was intravenously injected into the tail of each mouse, according to the method devised by Cunningham et al., the mouse was killed by vertebral dislocation and the spleen was placed in an Eagle's MEM medium maintained at 4°C. Next, the spleen was crushed between two slide glasses to prepare a splenic cell suspension and then it was filtered with a stainless mesh to prepare a monocellular suspension.

The antibody-production primary immune response of each group was examined by extracting spleens from all mice in the group to prepare a cell suspension and then counting the number of splenic cells forming hemolytic plaques. The experiment was performed three times for each group, The average number (mean±standard error) of splenic cells forming hemolytic plaques 4 days after the animal) was immunized with SRBC 11 days after being administered with the microbial cell preparation was obtained for each group.

Study group 197,880±28.900  $P > 0.01$

Control group 286,100±29.400

These results obviously indicate the immunological-competence-enhancing effect of oral administration of the S. lactis cell preparation of this invention.

(Case 10)

The arteriosclerosis-preventing effect of this agent will be described on the basis of the results of the following experiment.

We performed the following experiment in order to study the effect of SOD active S. lactis used in this invention in preventing pultaceous sclerosis resulting from aging.

Two groups each consisting of 15 Wistar rats (0 weeks old) and two groups each consisting of 15 SHR rats (10 weeks old) were used. The animals of one of the Wistar rat groups and one of the SHR rat groups used as control groups were allowed to take usual solid feed F2 (product of the Funabashi farm) and water freely and the animals of the other Wistar and SHR rat groups used as treated groups (study groups) were administered with pellets of F2 powder feed containing 10,000 ppm of the aforementioned S. lactis cell

15

preparation. The animals of the Wistar rate groups were kept until they became 27-month old and the animals of SHR rat groups were kept until they became 12-month old. The daily feed consumption was about 15-20 g for both groups and addition of the S. lactis preparation to the feed did not appear to affect its taste One week after the start of administration, pathohistological observation using a light microscope and an electron microscope detected no difference between the treated and the nontreated Wistar rat groups. But, when the animals of the Wistar rat groups became 27-month old. although sporadic alopecia and Kyphosis were observed in all cases in the nontreated Wistar rat group, these phenomena were not detected in the treated Wistar rat group. These results indicate that, while significant changes resulted from aging in the 27-month old rats of the nontreated Wistar rat groups, changes resulting from aging were inhibited in the treated Wistar rat group. In addition, when the animals of the Wistar rat groups became 6 month old and 27 month old and those of the SHR rat groups which generally have an only short life span became 6 month old and 12 month old, abnormal changes in the intima, the media and the adventita of each rat were observed by using a light microscope and an electron microscope.

The degrees of damage to endothelium, deposition of fat and protein-polysaccharide complex on endothelium appearance of chondrocyte-like cells and exposure of collagen fiber were observed for the intima, the degree of calcification was observed for the media and the degree of arteriolar constriction was observed for the adventitia (Table 4). The degree of these abnormal changes observed in each rat were evaluated on the basis of the average result for 5 specimens of the rat according to 5 stages. In the control groups, endothelium was maintained almost nomal and only slight expansion of microvillous projections was found by scanning-electromicroscopic observation. In contrast, in the nontreated group, fibrous hyperplasia on the segment of arterial wall, calcification of intimal stroma and appearance of chondrocyte-like spindle cells were observed. These abnormal changes were very similar in their structure to the cartilage matrix of animals in their senility. An obvious difference in the organic feature of arteriosclerosis was found between the administration group and the nontreated group. As compared with the administration group, severer obstructive changes of nutrition blood vessels in media and adventitia were observed in the control group. Biochemical tests found a significantly great amount of collagen, which indicates fibroplasia, in the nontreated group. In the study group, the increase of collagen was inhibited from an early stage (6-month old) (Table 5). Changes in amount of the collagen are in parallelism with those in the hydroxyproline.

We studied arterial changes resulting from aging in the above. The above results of the experiment indicate that the S. lactis cell preparation of this invention is effective and useful for preventing arteriosclerosis.

Table 4.  Arterial Changes Resulting from Aging

| Species | | Wistar | | | | | | SHR | | | |
|---|---|---|---|---|---|---|---|---|---|---|---|
| Observation period | | 1 week | | 6 months | | 27 months | | 6 months | | 12 months | |
| Group | | ☆ C | ☆☆ SL | C | SL | C | SL | C | SL | C | SL |
| Intima | a | − | − | + | + | 3+ | + | 2+ | + | 4+ | 2+ |
| | b | − | − | + | − | 2+ | + | + | + | 4+ | + |
| | c | − | − | 2+ | + | 4+ | 2+ | + | + | 5+ | 2+ |
| Media | d | − | − | + | + | 3+ | + | + | + | 4+ | + |
| Aventita | e | − | − | − | − | 2+ | + | + | + | 3+ | + |

☆C:              Control group

☆☆SL:         Group administered with S. lactis cell preparation

a:         Damage to endothelium and deposition of fat and
            protein-polysaccharide complex on endothelium

b:         Appearance of chondrocyte-like cells

c:         Exposure of collagen fiber

d:         Calcification

e:         Arteriolar constriction

−  :       Normal

+  :       Minor

2+ :       Moderate

3+, 4+, 5+:  Severe~Very severe

(Note)  Evaluated on the basis of the observation results of 5
            specimens for each rat.

Tagle 5.　Changes in the Amount of Hydroxyproline ( $\mu$g/DNA $\mu$g) in the Aorta and the Mesenteric Artery of SHR and Wistar Rats Resulting with Age.

| | | 80 days old | 6 months old | Senile (12 months old for SHR rats and 20 months old for Wistar rats) |
|---|---|---|---|---|
| Aorta | SHR (control group) | 6.12 ± 0.32 | 7.21 ± 0.35 | 8.90 ± 0.61 |
| | SHR (study group) | 6.05 ± 0.22 | 7.03 ± 0.33 | ☆ 7.15 ± 0.14 |
| | SHR (control group) | 2.99 ± 0.18 | 3.08 ± 0.09 | 7.35 ± 0.51 |
| Mesenteric artery | SHR (study group) | 3.05 ± 0.19 | 3.12 ± 0.02 | 7.05 ± 0.21 |
| | Wistar (control group) | 1.08 ± 0.06 | 1.65 ± 0.08 | 5.32 ± 0.64 |
| | Wistar (study group) | 1.80 ± 0.08 | 1.75 ± 0.12 | ☆☆ 3.93 ± 0.03 |

☆ P ＜0.05

☆☆ P＜0.01

The average results were obtained for groups consisting of 5 rats each.

(Case 11)

The anti-hypertensive effect of this agent will be described on the basis of the result of the following experiment. We confirmed the antihypertensive effect of this invention by orally administering a powder, granules, tables, capsules or a syrup which was prepared by combining a cell preparation of an SOD active strain of S. lactis used as the effective agent with auxiliary components such as a vehicle, a binder and a diluent and other components including $\beta$-carotene or mannitol if necessary. The dose of this agent should be varied according to age and body weight and it is usually desirable that the daily dose per adult of the S. lactis cell preparation for its oral administration be about 3-60 g.

In preferable examples of this invention, the medicine is in a form suitable for either administering all the daily dose at once or subdividing it into a few doses.

We performed the following experiment in order to study the effect of SOD active S. lactis used in this invention in preventing hypertension resulting from aging. Ten SHR rats with spontaneous hypertension were used as test animals and they were divided into two group each consisting of 5 animals. From the time when the rats were 10 weeks old until they became 12 months old, the rats of the control group (nontreated group) were allowed to take water and usual solid feed F2 (product of the Funabashi farm) freely and the rats of the study group were administered with pellets of F2 powder feed containing 10,000 ppm of the above S. lactis cell preparation. The daily feed consumption was about 15-20 g for both groups and addition of the S. lactis cell preparation to the feed did not appear to affect its taste. The systolic blood pressure of each rat was measured when it was 80 days old, 6 months old and 12 months old and the average blood pressure level measured at each time point was calculated for each group consisting of 5 rats. The blood pressure levels recorded for the rats of the control group when they were 80 days old ranged between 149-155 mmHg (average, 151.3 mmHg) and those recorded for the rats of the study group when they were 80 days old ranged between 142 mmHg and 148 mmHg (average, 145.8 mmHg). For the age of 80 days old, no significant difference was detected between the two groups. The blood pressure levels recorded for the rats of the control group when they were 6 months old ranged between 205 mmHg and 215 mmHg (average 208.4 mmHg) and those recorded for the rats of the study group when they were 6 months old ranged between 162 mmHg and 168 mmHg (average, 165.5 mmHg). For the age of 6 months old, Student's test found that the average blood pressure level of the study group was significantly lower than that of the control group (P<0.025). The blood pressure levels recorded for the rats of the control group when they were 12 months old ranged between 195 mmHg and 212 mmHg (average, 207.2 mmHg) and those recorded for the rats of the study group when they were 12 months old ranged between 70 mmHg and 175 mmHg (average, 172.8 mmHg). For the age of 12 months old which corresponds to the senile stage, the average blood pressure level of the study group was significantly lower than that of the control group (P<0.05).

The above experiment was performed in order to study the antihypertensive effect of a cell preparation of an SOD active strain of S. lactis. The results of this experiment indicate that this agent is effective and useful for preventing hypertension.

(Case 12)

The biological-response-modifying (BRM) effect of this agent will be explained on the basis of the results of the following experiment. We confirmed the BRM effect of this invention by orally or parenterally administering a powder, granules, tablets, capsules or a syrup which is prepared by combining a cell preparation of an SOD active strain of S. lactis used as the effective agent with auxiliary components such as vehicle, a binder and a diluant and other components according to necessity.

The dose of this agent should be varied according to age and body weight and it is usually desirable that the daily dose per adult of the S. lactis cell preparation for its oral administration be about 3 - 60 g.

In preferable examples of this invention, the medicine is in a form suitable for either administering all the daily dose at once or subdividing it into a few doses.

Six-week old male Balb/C and C3H mice were obtained from the Shizuoka test animal coorporative association and the following experiment was started when they were 7 to 9 weeks old.

1) The effect of the S. lactis cell preparation on Ehrlich's ascites carcinoma (dose dependence test)

Eighty Balb/C mice were used and $5 \times 10^4$ Ehrlich's ascites carcinoma cells were implanted into the ascites of each mouse. Twenty four hours after implantation, the mice were divided into four groups each consisting of 20 animals and 5 mg/kg, 15 mg/kg, 50 mg/kg or 150 mg/kg of the microbial cell preparation was intraperitoneally injected into each animal once daily successively for 7 days. The longer survival rate (%) of each animal was calculated 100 days after the implantation of carcinoma cells. The longer survival rates recorded for the groups admnistered with 5 mg/kg, 15 mg/kg, 50 mg/kg and 150 mg/kg of the microbial cell preparation were 9.5%, 85%, 100% and 60% respectively. It was concluded from these results that the optimum dose of the microbial cell preparation is 50 mg/kg and we decided to use this dose thereafter.

2) The effect of the S. lactis cell preparation on isologous tumors

$2 \times 10$ Meth A tumor cells were hypodermically implanted into each of 24 Balb.C mice and $2 \times 10^5$ MCA tumor cells were hypodermically implanted into each of 24 C3H mice. Twenty-four hours after implantation, 50 mg/kg of the microbial cell preparation was intraperitoneally injected to each of 12 Balb/C and 12 C3H mice once daily successively for 7 days. Each of the other 12 Balb/C and 12 C3H mice constituting control groups was administered with the same volume as the microbial cell preparation of a mixture of isotonic saline and phosphate buffer in the same manner as above.

Twenty-one days after implantation, the tumor was cut off and its weight was measured. The recorded average weight of the Meth A tumors of mice receiving administration of the microbial cell preparation was 51% of. that for the Balb/C control group. The recorded average weight of the MCA tumors of animals receiving administration was 50% of that for the C3H control group.

The above two experiments were performed in order to study the (BRM) effect of a cell preparation of and SOD active strain of S. lactis. The results of these experiments indicate that this agent has a superior antineoplastic effect.

(Case 13)

We performed the following experiment in order to study the toxicity of cis-DDP.

Daily doses of 2 mg/kg, 3 mg/kg and 4.5 mg/kg body weight of cis-DDP were intraperitoneally administered to male DDY mice (6 weeks old) successively for 5 days. The nontreated group and the 2 mg/kg group consisted of 20 animals each. Three, 5, 7 and 17 days after the end of administration, blood and urine samples were collected from each animals in the 2 mg/kg group. Blood urea nitrogen(BUN) levels, serum creatinine levels and urine N-acetyl-$\beta$-D-glucosaminase (NAG) activity levels were determined for these samples by conventional methods in order to observe abnormal changes resulting from the toxicity of cis-DDP. Although similar BUN levels and similar serum creatinine levels were recorded for the nontreated group and the 2 mg/kg group, the BUN level and the serum creatinine level changed as the dose of cis-DDP increased. This indicated that the toxicity of cis-DDP is dose-dependent. Therefore, the dose of cis-DDP for producing toxicity was determined to be 3.5 mg/kg. The dose of cis-DDP used in the following experiments was 3.5 mg/kg.

(Case 14)

We studied the influence of administration on the body weights of test animals. Jointly with administration of cis-DDP, a daily dose of 3,500 units of SOD (Sigma company, Type III, 3,000 units/mg) was intraperitoneally administered to each of 20 mice successively for 5 days and a daily dose of 15 mg of the S. lactis cell preparation was orally administered to each of other 20 mice successively for 7 days. A daily dose of 15 mg of the S. lactis cell preparation was administered to each of other 22 mice from five days before administration of cis-DDP was started. Twenty mice receiving no administration, 20 mice administered with cis-DDP alone and 20 mice administered with the S. lactis cell preparation alone successively for 12 days were used as control groups. Changes in the body weight of each animal in the study groups and the control groups were examined. No significant difference in the average body weight measured before administration or the average body weight measured 5 days after the last administration was detected between any two of the animal groups.

(Case 15)

We studied the effect of joint use of cis-DDP and SOD and that of joint use of cis-DDP and S. lactis on mice with carcinoma. Six-week old male mice of the ICR species were used and $1.5 \times 10^6$ Ehrlich carcinoma cells were implanted into the femoral muscle of each mouse. The test animals were divided into a nontreated group consisting of 15 mice, a cis-DDP group consisting of 15 mice, a group consisting of 12 mice which were jointly administered with cis-DDP and SOD and a group consisting of 15 mice which were jointly administered with cis-DDP and the S. lactis cell preparation. There were many death cases and only two mice remained alive in the group administered with 3.5 mg/kg of cis-DDP alone. All cases remained alive in the nontreated tumor-bearing group. It is thought from these results that the death cases observed in the group administered with cis-DDP alone resulted from the toxicity of cis-DDP. In contrast, reduced tumor weights were recorded for the cis-DDP plus SOD joint administration group and the cis-DDP plus cell-preparation joint administration group and the death rates recorded for these groups were 6/15 and 0 respectively.

Both an antineoplastic effect and reduced toxicity of cis-DDP were achieved by joint administration. One death case in the S. lactis cell preparation group resulted from miss-administration of the preparation into the windpipe.

Table 6.   The Effect of Superoxide Dismutase (SOD) and S. Lactis cell Preparation (SL) on cis-DDP in Mice [a]

| Treatment | | Mouse, Number | Tumor weight (g) |
|---|---|---|---|
| ( − ) | | 15 | $1.29 \pm 0.638$ |
| cis-DDP | b) | 2 | $0.18 \pm 0.248$ |
| cis-DDP + SOD | c) | 6 | $0.58 \pm 0.221$ |
| ( − ) SOD | | 12 | $1.73 \pm 1.051$ |
| cis-DDP + SL | d) | 12 | $0.66 \pm 0.321$ |
| ( − ) SL | | 14 | $1.58 \pm 0.925$ |

a)      Ehrlich carcinoma cells were implanted into the left femoral muscles of ICR mice and the weght of the tumor was measured 20 days after implantation.

b)      Intraperitoneal administration of 3.5 mg/kg of cis-DDP was performed once daily for 5 days from 24 hours following tumor implantation.

c)      Intraperitoneal administration of SOD (3,500 units/mouse) was performed one hour after DDP administration.

d)      Oral administration of SL (15 mg/mouse) wqas performed once daily for 13 days from 6 days before tumor implantation.

(Case 16)

We studied the pathological effect of cis-DDP administration on renal tissue. After the end of case 15, the animals of each group were killed and their fresh kidney, liver and spleen were fixed with Bouin's fixative. Next, paraffin sections were prepared from the fixed organs and then samples for light-microscopic observation were prepared from the parafin sections by hematoxylin-eosin stain. PAS-H and PAS-AB (pH 2.5).

Separately, after a small piece of renal tissue was preliminarily fixed with a 1.5% glutaraldehyde solution in cacodylate buffer solution (pH 7.3), the tissue piece was fixed with a 1% osmic acid solution in the above buffer solution and then the fixed piece was washed with the above buffer solution before dehydration prior to embedding the dehydrated tissue piece in Epon 81. Then, ultrathin sections of 100 mm thickness were prepared using an LKB ultratome. These sections were electron-microscopically observed after being stained with lead citrate or uranium acetate. Normal electron-microscopic images were observed for all the cortex, the medulla and the papilla renalis of the renal tissue of the nontreated group. In the group administered with 3.5 mg/kg of cis-DDP successively for 5 days, electron-microscopic observation performed 5 days after the last administration found accumulation of many hyaline casts in the cortical region mainly in distal urinary tubules, and also found necrotic images of distal urinary tubules in some cases. Although it appeared that almost normal images of Malpighian corpuscles and proximal urinary tubules were observed, the PAS staining of brush border membrane exhibited a slight positive result which suggested liquefaction of glycocarics. In the medullary region, hyaline casts significantly appeared mainly in the collecting tubules of distal urinary tubules which resulted in increased obstruction of ureters reaching the papilla renalis. Extension of distal urinary tubules and squamatization of urinary tubules and epithelium which appeared to have resulted from obstructive disorder were observed.

In the group jointly administered with cis-DDP and SOD, generally only a slight degree of ureteral-obstructive failure resulted from administration and great reduction of functional failure was achieved by SOD. Pathological results obtained for the group jointly administered with cis-DDP and the S. lactis cell preparation were similar to those obtained for the group jointly administered with cis-DDP and SOD.

The above degrees of failure are indicated by the frequency of hyaline cast appearance in Table 2. In the positive control groups which consist of the group jointly administered with cis-DDP and SOD, the group jointly administered with cis-DDP and the S. lactis cell preparation and the group preliminarily administered with the cell preparation before cis-DDP administration, a significant reduction of toxicity was achieved as compared to the group administered with cis-DDP alone. The above accumulation of hyaline casts recorded for the joint administration groups was not so significant as was indicated by the figure (Table 7) in many cases.

Table 7. The Effect of Superoxide Dismutase (SOD) and S. Lactis Cell Preparation (SL) on the Formation of Hyaline Casts [a] During Administration of cis-DDP

| Treatment | Frequency of appearance (number of times ± standard error) | | |
| --- | --- | --- | --- |
| | Cortex | Medulla | Papilla renalis |
| Control | 0 ± 0 | 0 ± 0 | 0.1 ± 0.3 |
| Cis-DDP | 119 ± 59 | 51 ± 25 | 25 ± 18 |
| Cis-DDP + SOD | 26 ± 13 | 10 ± 6 | 9 ± 8 |
| Cis-DDP + SL | 87 ± 46 | 29 ± 17 | 10 ± 8 |
| SL + Cis-DDP + SL | 59 ± 37 | 25 ± 14 | 9 ± 5 |

a) Abnormal changes in distal and proximal urinary tubules
b) Mean ± standard error

(Case 17)

We performed the following animal experiment in order to study the radioprotective effect of the SOD active S. lactis cell preparation of this invention. Five to six weeks old female C3H/He SLC mice (SPF) were used as test animals. Six groups each consisting of 7-9 mice were used according to the following experimental design (Table 8). These six groups consisted of 3 non-radiation groups and 3 radiation groups irradiated with 300 rads over the whole body. The amount of a suspension of the S. lactis cell preparation (hereafter referred to as SL) in isotonic saline solution for each administration using a stomach tube was established as 0.5 ml (10 mg SL/mouse).

The 3 non-radiation groups consisted of a group receiving no administration, a group once administered with the suspension before irradiation and a group administered with the suspension three times on alternate days. The 3 radiation groups consisted of a group receiving no administration, a group once administered with the suspension before irradiation and a group administered with the suspension three times on alternate days. Other 6 groups were used to study the primary immune response of animals administered with SL before irradiation. Sheep red blood cells stored in an Alserver liquid were used and they were washed with phosphate buffer saline (PBS) three times immediately prior to use. Each mouse in these 6 groups was immunized with SRBC by intravenously injecting 0.2 ml of a 25% SPBC suspension into the tail. According to the method devised by Cunningham et al., the mouse was killed by vertebral dislocation and the spleen was placed in an Eagle's MEM medium maintained at 4 °C. Next, the spleen was crushed between two slide glasses to prepare a splenic cell suspension and then it was filtered with a stainless steel mesh to prepare a monocellular suspension. The antibody-producing primary immune response of each group was examined by extracting spleens from all mice in the group to prepare a cell suspension and then counting the number of splenic cells forming hemolytic plaques. The experiment was performed three times for each group and the average number (mean± standard error) of splenic cells forming hemolytic plaques was obtained for each group (Figs. 10 and 11).

The animals of each of the 6 groups not administered with SRBC were serobiochemically and pathologically examined. Specifically, the whole blood of each mouse was collected and the number of white blood cells, the number of red blood cells and GOT, GPT, γ-GTP, alkaline phosphatase, Na and K levels were determined for the collected blood. Pathological samples were prepared from the liver, the spleen, the small intestine and the cervical lymph nodes of each mouse. An x-ray irradiator of the ML-3 type manufactured by Mitsubishi Electric Company was used for irradiation and each mouse was irradiated at a voltage of 3 MV and a dose rate of 100 rads by the above device which was located at a 80 cm distance. As mentioned above, the dose of irradiation was 300 rads for all cases.

Table 8. Experimental Design for Measuring the Effectiveness of S. Lactis Cell Preparation for Radio-protective Effect

| group | Prior day 7 to irradiation | Prior day 1 to irradiation | irradiation | Post day 10 to irradiation | Post day 14 to irradiation |
|---|---|---|---|---|---|
| 1 | | | OR | SRBC | PEC test |
| 2 | | SL | OR | SRBC | PEC test |
| 3 | SL    SL | SL | OR | SRBC | PEC test |
| 4 | | | 300R | SRBC | PEC test |
| 5 | | SL | 300R | SRBC | PEC test |
| 6 | SL    SL | SL | 300R | SRBC | PEC test |

Notes) SL: S. lactis cell preparation

R: rad

SRBC: Immunization with a sheep red blood cell suspension

PFC test: Counting the number of splenic cells forming hemolytic plagues

## Claims

1. An SOD (superoxide dismutase) agent consisting of or derived from a culture of an SOD-active strain selected from the Streptococcus lactis group to grow on a particular culture medium.

24

2. A method of manufacturing an SOD agent, comprising the steps of:

inoculating an SOD-active strain selected from the <u>Streptococcus lactis</u> group on a specific culture medium;

allowing the SOD-active strain to grow under a controlled constant temperature condition;

subjecting the culture obtained from the preceding step to the centrifugal separating process;

washing the culture obtained from the centrifugal separation; and, if necessary,

disrupting the purified product by any appropriate means.

3. A method of manufacturing an SOD agent as defined in Claim 2, wherein the culture medium contains 1% glucose, 0.8% peptone, 0.4% yeast extract, 0.3% sodium citrate, 0.25% sodium bicarbonate, and for the rest water.

4. A method of manufacturing an SOD agent as defined in Claim 2, where the growth of the SOD-active strain occurs for a time period of between 24 hours and 30 hours.

5. A method according to any of Claims 2 to 4 in which the centrifugal separating process for the culture of the SOD-active strain of <u>S. lactis</u> takes place for a time period of between 10 min. and 30 min. at 10,000 rpm.

6. A method according to any of Claims 2 to 5 in which the purifying process followed by the disrupting process includes the steps of washing the culture obtained from the preceding step with a sterilized physiological saline for three to five times, and of disrupting the washed culture by means of ultrasonic irradiation, application of high-pressure, or by using beads as appropriate, if necessary.

7. A composition for inhibiting senile changes on aging, containing the SOD-active strain from the <u>streptococcus lactis</u> group or an SOD-containing isolate therefrom, together with a pharmaceutical carrier or excipient.

8. A composition for ameriolating the side effects by a superoxide producing chemotherapeutic agent, containing the SOD-active strain selected from the <u>Streptococcus lactis</u> group or an SOD - containing isolate therefrom together with a pharmaceutical carrier or excipient.

9. A radioprotective composition containing the SOD-active strain selected from the <u>Streptococcus lactis</u> or an SOD - containing isolate therefrom together with a pharmaceutical carrier or excipient.

10. The SOD-active strain as defined in any of the preceding claims is an <u>S. lactis</u>.

11. The use of a culture of <u>Streptococcus lactis</u> or an SOD - containing isolate therefrom in the preparation of a pharmaceutical composition for treatment of superoxide - induced disease.

# FIG. 1

wave length

Infrared   Absorption   Spectrum

0 289 667

F I G. 2

(−)                                              (+)

human SOD

S.Lactis SOD

indicates the band (including SOD) not responsive to NBT

# FIG. 3

(a)

(b)

# F I G. 4.

Relative absorption intensity of fluorescence

40 ☐ For group of rats fed s.lactis
▨ For group of rats not fed s.lactis
30
20
10

serum          liver          brain

# FIG. 5

←Fed

←Not fed

# FIG. 6

Fed                    Not fed

# FIG. 7

Fed                    Not fed

## F I G. 8

Fed                    Not fed

## F I G. 9

Fed                    Not fed

# F I G. 10

# F I G. 11

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| X | CHEMICAL ABSTRACTS, vol. 104, no. 15, April 1986, page 561, abstract no. 128168u, Columbus, Ohio, US; O. HOLST et al.: "Continuous culture with complete cell recycle to obtain high cell densities in product inhibited cultures; cultivation of Streptococcus lactis for production of superoxide dismutase", & APPL. MICROBIOL. BIOTECHNOL. 1985, 23(1), 10-14 * Abstract * | 1,6 | C 12 N 9/02 A 61 K 37/50 |
| X | CHEMICAL ABSTRACTS, vol. 105, no. 1, July 1986, page 496, abstract no. 5096g, Columbus, Ohio, US; L. HANSSON et al.: "Cultivation of Saccharomyces cerevisiae and Streptococcus lactis for production and extraction of the intracellular superoxide dismutase (SOD) enzymes", & EUR. CONGR. BIOTECHNOL., 3rd 1984, 1, 117-21 * Abstract * | 1,6 | |
| X | CHEMICAL ABSTRACTS, vol. 93, no. 1, 7th July 1980, page 273, abstract no. 2804j, Columbus, Ohio, US; T. KIRBY: "Distinguishing between manganese-containing and iron-containing superoxide dismutases in crude extracts of cells", & ARCH. BIOCHEM. BIOPHYS. 1980, 201(2), 551-5 * Abstract * | 1,6 | **TECHNICAL FIELDS SEARCHED (Int. Cl.4)**<br><br>C 12 N<br>A 61 K |
| Y | EP-A-0 172 577 (TAKEDA) * Claims; pages 2-4 *<br>---     -/- | 1,6 | |

The present search report has been drawn up for all claims

| Place of search | Date of completion of the search | Examiner |
|---|---|---|
| THE HAGUE | 09-12-1987 | DELANGHE L.L.M. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent
Office

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| E | CHEMICAL ABSTRACTS, vol. 107, no. 13, 28th September 1987, page 67, abstract no. 109347x, Columbus, Ohio, US; & JP-A-62 103 022 (NIKKEN FOOD CO., LTD) 13-05-1987 | 1-11 | |

TECHNICAL FIELDS
SEARCHED (Int. Cl.4)

The present search report has been drawn up for all claims

| Place of search | Date of completion of the search | Examiner |
|---|---|---|
| THE HAGUE | 09-12-1987 | DELANGHE L.L.M. |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P0401)